(19) Europäisches Patentamt
European Patent Office
Office européen des brevets

(11) Veröffentlichungsnummer : **0 081 745**
**B1**

(12) # EUROPÄISCHE PATENTSCHRIFT

(45) Veröffentlichungstag der Patentschrift : **30.10.85**.

(51) Int. Cl.⁴ : **A 61 K 7/13**// C07D213/69

(21) Anmeldenummer : **82111092.1**

(22) Anmeldetag : **01.12.82**

(54) **Haarfärbemittel, enthaltend 5-Halo-2,3-pyridindiole als Kupplerkomponente.**

(30) Priorität : **09.12.81 DE 3148651**

(43) Veröffentlichungstag der Anmeldung : **22.06.83 Patentblatt 83/25**

(45) Bekanntmachung des Hinweises auf die Patenterteilung : **30.10.85 Patentblatt 85/44**

(84) Benannte Vertragsstaaten : **AT BE CH DE FR GB IT LI LU NL SE**

(56) Entgegenhaltungen :
**DE-A- 1 695 035**
**DE-A- 2 629 805**
**DE-A- 2 739 227**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Rose, David, Dr.**
**Holbeinweg 7**
**D-4010 Hilden (DE)**
Erfinder : **Maak, Norbert, Dr.**
**Liebigstrasse 18**
**D-4040 Neuss (DE)**

**EP 0 081 745 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Verwendung von 5-Halo-2,3-pyridindiolen als Kupplerkomponenten in Oxidationshaarfarben.

Für das Färben von Haaren werden meist sogenannte Oxidationshaarfarben verwendet, die durch oxidative Kupplung einer Entwicklerkomponente mit einer Kupplerkomponente entstehen, da diese sehr intensive Farben mit befriedigenden Echtheitseigenschaften ergeben. Gute Oxidationshaarfarben müssen folgende wesentliche Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen auch ein gutes Aufziehvermögen auf menschlichem Haar besitzen und sollen in toxikologischer und dermatologischer Hinsicht unbedenklich sein.

Bisher wurden als Kupplerkomponenten vor allem m-Phenylendiaminderivate, Phenole, Naphthole, Resorcin-Derivate oder Pyrazolone verwendet.

5-Chlor-2.3-pyridindiol ist aus DE-A-16 95 035 bekannt und die Verwendung von 5-Halo-2.3-pyridindiolen zur Herstellung von Azofarbstoffen ist aus DE-A-27 39 277 bekannt. In DE-A-26 29 805 wird die Verwendung von Pyridindiolen als Kupplerkomponente für Oxidationshaarfärbemittel beschrieben. Die aus dieser Druckschrift bekannten Pyridindiole führen zu Haaranfärbungen von unbefriedigender Reibechtheit.

Aufgabe der vorliegenden Erfindung ist es eine weitere Kupplerkomponente zur Verfügung zu stellen, die die vorgenannten Voraussetzungen in optimaler Weise erfüllen kann und es ermöglicht, neue Farbnuancen bereitzustellen.

Es wurde nun überraschend gefunden, daß man zu Oxidationshaarfarben, die den genannten Anforderungen in besonders hohem Maße entsprechen, gelangt, wenn man als Kupplerkomponente 5-Halo-2,3-pyridindiole verwendet.

Gegenstand der vorliegenden Erfindung sind Haarfarbstoffe, welche eine Verbindung der allgemeinen Formel

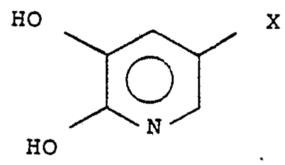

wobei X für ein Chlor- oder Bromatom steht, enthalten. Diese 5-Halo-2,3-pyridindiole ergeben mit einer Vielzahl von Entwicklersysteme Farben von besonderer Intensität, vor allem im Rot- bis Rubinbereich. Diese Haarfarben zeichnen sich durch gute Lichtechtheitseigenschaften, Wärmestabilität aus. Sie sind toxikologisch und dermatologisch unbedenklich. Die erfindungsgemäßen Kupplerkomponenten können als solche oder auch in Form ihrer Salze mit anorganischen oder organischen Säuren eingesetzt werden.

Für die erfindungsgemäßen Kuppler sind praktisch alle an sich bekannten Entwicklersysteme geeignet. Bevorzugt werden aromatische und heterocyclische Diamine wie z. B. 2,4,5,6-Tetraaminopyrimidin, p-Phenylendiamin, p-Toluylendiamin, 2-Chlor-p-phenylendiamin, heterocyclische Hydrazone und Diamino-pyridine. Die erfindungsgemäßen Kupplersubstanzen und die genannten Entwicklersubstanzen sind literaturbekannt. Die Herstellung von 5-Chlor-2,3-pyridindiol ist z. B. in der DE-A-16 95 035 beschrieben.

In den erfindungsgemäßen Haarfärbemitteln werden die Kupplerkomponenten im allgemeinen in etwa molaren Mengen, bezogen auf die verwendeten Entwicklersubstanzen, eingesetzt. Grundsätzlich ist jedoch auch ein gewisser Überschuß an Kupplerkomponente oder Entwicklerkomponente nicht nachteilig.

In den erfindungsgemäßen Haarfärbemitteln können noch weitere bekannte Kupplersubstanzen anwesend sein u. es können auch Gemische von bekannten Entwicklerkomponenten eingesetzt werden.

Darüber hinaus können die erfindungsgemäßen Haarfärbemittel auch übliche direktziehende Farbstoffe enthalten, falls dies zur Erzielung besonderer Farbnuancen zweckmäßig ist.

Die oxidative Kupplung, d. h. die Entwicklung der Färbung, kann grundsätzlich wie bei ansich bekannten Oxidationshaarfarbstoffen, durch Luftsauerstoff erfolgen. In der Regel werden jedoch chemische Oxidationsmittel eingesetzt. Als solche kommen insbesondere Wasserstoffperoxid oder dessen Anlagerungsprodukte an Harnstoff, Melamin oder Natriumborat in Betracht.

Die erfindungsgemäßen Haarfärbemittel werden für den Einsatz in entsprechende kosmetische Zubereitungen, wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färbender Zubereitungen an Kuppler-Entwicklerkombination beträgt 0,2 bis 5 Gewichtsprozent, vorzugsweise 1-3 Gewichtsprozent, bezogen auf die gesamte Zubereitung. Zur Herstellung von Cremes, Emulsionen oder Gelen werden die Farbstoffkomponenten mit üblichen Träger- und Hilfsstoffen gemischt. Als solche sind z. B. Netz- oder Emulgiermittel vom anionischen oder

nichtionogenen Typ, wie Alkylbenzolsulfonate, Fettalkoholsulfate, Alkylsulfonate, Fettsäurealkanolamide, Anlagerungsprodukte von Ethylenoxid an Fettalkohole, Verdickungsmittel, wie Methylcellulose, Stärke, höhere Fettalkohole, Paraffinöl, Fettsäuren, Duftstoffe und Haarpflegemittel, wie Pantothensäure oder Cholesterin zu nennen.

Die Anwendung der erfindungsgemäßen Haarfärbemittel erfolgt, unabhängig von der Applikationsform, im schwach sauren, neutralen, vorzugsweise alkalischen Milieu bei einem pH-Wert von 8-10. Die Anwendungstemperaturen sollen im Bereich von 15 bis 40 °C liegen. Man läßt die Haarfärbemittel etwa 30 Minuten einwirken ; anschließend werden sie vom zu färbenden Haar mit Wasser ausgespült und das Haar mit einem milden Shampoo nachgewaschen und getrocknet.

Die nachfolgenden Beispiele sollen den Erfindungsgegenstand näher erläutern :

## Beispiele

Die erfindungsgemäßen Haarfärbemittel wurden in Form einer Creme-Emulsion eingesetzt. Dazu wurden jeweils 0,01 Mol der in der nachstehenden Tabelle aufgeführten Kuppler- und Entwicklerkomponenten in

10 Gew.-Teilen Fettalkoholen der Kettenlänge $C_{12}$-$C_{18}$
10 Gew.-Teilen Fettalkoholsulfat (Natriumsalz) der Kettenlänge $C_{12}$-$C_{18}$
75 Gew.-Teilen Wasser

eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 3 %iger Wasserstoffperoxidlösung als Oxidationsmittel durchgeführt. Die jeweilige Färbecreme mit dem zugefügtem Oxidationsmittel wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind in nachstehender Tabelle aufgeführt.

Tabelle 1

| Farbstoffkomponenten<br>a) Entwickler | <br>b) Kuppler | Nuance des gefärbten Haares 3%ige $H_2O_2$-Lösung |
|---|---|---|
| 1) p-Phenylendiamin | 5-Chlor-2,3-pyridindiol | violettbraun |
| 2) N,N-Dimethyl-p-phenylendiamin | " " | dunkelrubin |
| 3) N-Butyl-N-sulfobutyl-p-phenylendiamin | " " | graubraun |
| 4) 2-Chlor-p-phenylendiamin | " " | rotbraun |
| 5) N-Ethyl-N-ß-hydroxyethyl-p-phenylendiamin | " " | dunkelrubin |
| 6) 2-Methylamino-4,5,6-triaminopyrimidin | " " | braunorange |
| 7) 2,5-Diaminopyridin | " " | rotbraun |
| 8) N-2-Hydroxypropyl-p-phenylendiamin | " " | rotbraun |
| 9) N-Methyl-p-phenylendiamin | " " | violettbraun |
| 10) 2,4,5,6-Tetraaminopyrimidin | " " | graubraun |
| 11) p-Toluylendiamin | " " | rotbraun |
| 12) N-(p-Aminophenyl)-N',N'-bis-(ß-hydroxyethyl)-1,3-diaminopropan | " " | violettbraun |
| 13) p-Toluylendiamin | 5-Brom-2,3-pyridindiol | rotbraun |
| 14) 2,4,5,6-Tetraaminopyrimidin | " " | graubraun |

...

**Patentansprüche**

1. Haarfärbemittel auf Basis von Oxidationsfarbstoffen, dadurch gekennzeichnet, daß sie 5-Halo-2,3-pyridindiole der allgemeinen Formel

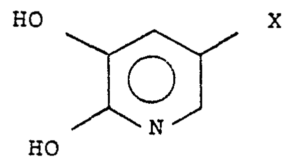

wobei X ein Chlor- oder Bromatom bedeutet, als Kupplerkomponente neben den in Oxidationshaarfarben üblichen Entwicklersubstanzen und Träger- und Hilfsstoffen enthält.

2. Haarfärbemittel nach Anspruch 1, gekennzeichnet durch einen Gehalt an Entwickler-Kuppler-Kombination von 0,2 bis 5 Gew.-%, vorzugsweise 1-3 Gew.-%, bezogen auf das gesamte Mittel.

**Claims**

1. Hair dyes based on oxidation dyes, characterized in that they contain 5-halo-2,3-pyridine diols corresponding to the following general formula

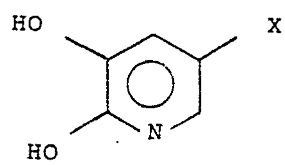

in which X is a chlorine or bromine atom, as coupler component in addition to the developer substances and carriers and auxiliaries normally used in oxidation hair dyes.

2. Hair dyes as claimed in Claim 1, characterized by a content of developer-coupler combination of from 0.2 to 5 % by weight and preferably of from 1 to 3 % by weight, based on the dye as a whole.

**Revendications**

1. Agent de teinture pour cheveux à base de colorants d'oxydation, caractérisé en ce qu'il contient des 5-halo-2,3-pyridine-diols de formule générale

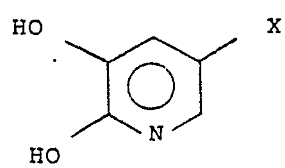

dans laquelle X signifie un atome de chlore ou de brome, comme composant de couplage à côté des substances de développement et des substances de support et auxiliaires en usage dans les teintures d'oxydation pour cheveux.

2. Agent de teinture pour cheveux selon la revendication 1, caractérisé par une teneur en la combinaison agent de développement-coupleur de 0,2 à 5 % en poids, de préférence de 1 à 3 % en poids par rapport à l'agent total.